# EUROPÄISCHE PATENTSCHRIFT

(11) **EP 3 500 674 B1**
(45) Veröffentlichungstag und Bekanntmachung des Hinweises auf die Patenterteilung: **14.10.2020**
(21) Anmeldenummer: 17761794.1
(22) Anmeldetag: 02.08.2017
(51) Int. Cl.: C12P 7/46, C12N 15/52, C12N 9/00, C12N 9/02, C12N 9/04, C12N 9/12, C12N 9/88, C07K 14/38, C07K 14/39, C07K 14/395

(54) **GENTECHNISCH VERÄNDERTE HEFE ZUR FERMENTATION VON GLYCEROL**
GENETICALLY MODIFIED YEAST FOR FERMENTING GLYCEROL
LEVURE GÉNÉTIQUEMENT MODIFIÉE POUR LA FERMENTATION DU GLYCÉROL

(30) Priorität: 19.08.2016 DE 102016115425
(43) Veröffentlichungstag der Anmeldung: 26.06.2019
(73) Patentinhaber: Jacobs University Bremen gGmbH, 28759 Bremen (DE)
(72) Erfinder: NEVOIGT, Elke, 10439 Berlin (DE)
(74) Vertreter: Weidner Stern Jeschke
(86) Internationale Anmeldenummer: PCT/DE2017/100655
(87) Internationale Veröffentlichungsnummer: WO 2018/033176

(56) Entgegenhaltungen:
- EP-A1- 2 495 304
- WO-A1-2009/065778
- WO-A1-2010/051324
- WO-A1-2013/004670
- WO-A1-2016/008819
- AHN J. H. ET AL: "Production of succinic acid by metabolically engineered microorganisms", CURRENT OPINION IN BIOTECHNOLOGY, Bd. 42, 15. März 2016 (2016-03-15), Seiten 54-66, XP029831135, ISSN: 0958-1669, DOI: 10.1016/J.COPBIO.2016.02.034

## Beschreibung

Die Erfindung betrifft eine gentechnisch veränderte Hefe zur Fermentation von Glycerol.

Die Bäckerhefe (*Saccharomyces cerevisiae*) ist ein beliebtes Objekt des Metabolic Engineering sowie ein attraktiver Produktionsorganismus in der industriellen Biotechnologie. Es wurden zahlreiche Ansätze verfolgt, um diesen Organismus zur Herstellung von industriell relevanten Produkten wie Biokraftstoffen, Grund- und Feinchemikalien (einschließlich Arzneimitteln) sowie Proteinwirkstoffen nutzbar zu machen (Borodina & Nielsen, 2014; Hong & Nielsen, 2012; Nevoigt, 2008).

*S. cerevisiae* wächst nur schlecht auf Glycerol als Kohlenstoffquelle. Zwei Studien haben gezeigt, dass die in der Forschung und industriell häufig verwendeten S. *cerevisiae-*Stämme die Zugabe von Ergänzungsstoffen (wie Aminosäuren und Nukleobasen) erfordern, um auf Glycerol zu wachsen (Merico et al 2011; Swinnen et al., 2013). Lediglich einzelne Isolate sind in der Lage, Glycerol ohne solche Medienzusätze als Kohlenstoffquelle zu nutzen (Swinnen et al. 2013).

Ein wesentlicher Vorteil bei der Nutzung von Glycerol als einziger oder zusätzlicher Kohlenstoffquelle in der industriellen Biotechnologie besteht darin, dass der Glycerol-Katabolismus im Vergleich zu Glucose pro C-Mol mehr Reduktionsäquivalente liefert. Dies ermöglicht grundsätzlich höhere maximale theoretische Ausbeuten von Stoffwechselprodukten, deren Syntheseweg Reduktionsäquivalente wie beispielsweise NADH oder NADPH erfordert. Ein Beispiel für ein solches Stoffwechselprodukt, das gleichzeitig von hohem kommerziellen Interesse ist, ist Bernsteinsäure (Butandisäure; McKinlay et al., 2007).

Zur Metabolisierung muss Glycerol zunächst in die Zelle aufgenommen werden. Bei Mikroorganismen sind grundsätzlich zwei Arten von Transportsystemen gefunden worden (s. Fakas et al. 2009 und Lin et al. 1976). Der erste Typ beruht auf erleichterter Diffusion, benötigt keine Energie und hängt von Glycerol-Facilitatoren ab. Im Gegensatz dazu ist der zweite Typ ein energieabhängiger aktiver Transport auf Basis eines Glycerol/H⁺-Symporters. Es war lange umstritten, wie Glycerol in die S. *cerevisiae* Zelle transportiert wird (Neves et al. 2004). Schließlich hat sich gezeigt, dass ein durch *STL1* codiertes aktives Transportsystem von entscheidender Bedeutung ist (Ferreira et al. 2005). Ein von *FPS1* codierter Glycerol-Facilitator ist natürlichweise auch in *S*. *cerevisiae* vorhanden (Luyten et al., 1995), scheint bei dieser Art aber keine entscheidende Rolle für die Glycerol-Aufnahme zu spielen. Letzteres belegt u.a. die Tatsache, dass die Deletion von *FPS1* das Wachstum in Glycerol-haltigem Medium nicht negativ beeinträchtigt (Tämas et al. 1999).

Generell erfolgt der initiale Abbau von Glycerol in Mikroorganismen auf drei Wegen (s. Fakas et al. 2009). Der Glycerol-3-Phosphat-Weg (auch G3P-Weg oder Phosphorylierungsweg) führt über die Phosphorylierung von Glycerol durch die Glycerolkinase zu L-Glycerol-3-phosphat (G3P), welches durch die mitochondriale FAD-abhängige Glycerol-3-phosphat-Dehydrogenase zu Dihydroxyacetonphosphat (DHAP) oxidiert wird. Der DHA-Weg (auch Oxidationsweg) führt über die durch Glyceroldehydrogenase katalysierte Oxidation von Glycerol zu Dihydroxyaceton (DHA), welches von der Dihydroxacetonkinase zu Dihydroxyacetonphosphat (DHAP) phosphoryliert wird. Der Glycerinaldehyd-Weg führt über Glycerinaldehyd zu Glycerinaldeyd-3-Phosphat. Sowohl DHAP als auch Glycerinaldeyd-3-Phosphat sind Zwischenprodukte der Glykolyse, dem zentralen Abbauweg im Kohlenhydratstoffwechsel der Hefe *S*. *cerevisiae.*

Experimentelle Daten deuten darauf hin, dass *S*. *cerevisiae* Glycerol ausschließlich über den Phosphorylierungsweg, d.h. über L-Glycerin-3-phosphat, abbaut, d.h. lediglich die von *GUT1* und *GUT2* codierten Enzyme Glycerolkinase und Glycerol-3-Phosphat-Dehydrogenase verwendet. Experimentelle Beweise für die Bedeutung von Gut1 und Gut2 für das Wachstum von *S. cerevisiae* auf Glycerol wurden erstmalig von Sprague und Cronan (1977) bereitgestellt. Deren Studien wurden in Medien durchgeführt, die komplexe Zusätze wie mehrere Aminosäuren und Nukleobasen enthielt. Swinnen et al. 2013 konnten allerdings zeigen, dass die Gene *STL1, GUT1* und *GUT2* auch eine entscheidende und exklusive Rolle für das Wachstum von *S. cerevisiae* in Glycerol-haltigem Medium ohne Zusätze haben.

Es ist unklar, ob der oxidative Glycerol-Abbauweg über DHA Weg in *S. cerevisiae* natürlicherweise funktional ist. In der Tat zeigten weder eine *gut1-* noch eine *gut2-*Deletionsmutante signifikantes Wachstum auf Glycerol (Swinnen et al. 2013), ein Ergebnis, das eine wichtige Rolle des DHA-Weges für diese Funktion ausschließt. Darüber hinaus konnten weder Norbeck und Blomberg (1997) noch Nguyen und Nevoigt (2009) eine native Glycerol-Dehydrogenase-Aktivität in *S. cerevisiae* messen. Im Gegensatz dazu schlagen andere Autoren vor, dass Glycerol durch eine NADP⁺-abhängige Glycerol-Dehydrogenase (GDH) zu Dihydroxyaceton umgewandelt wird, wobei die *Gene ARA1, GCY1, GRE3* und *YPR1* beteiligt sein sollen (Izawa et al., 2004). Zwar gibt es Zweifel an der Existenz einer natürlichen Glyceroldehydrogenase in *S. cerevisiae,* eine messbare Aktivität des zweiten Enzyms des DHA-Wegs, der durch die Gene *DAK1* und *DAK2* codierten Dihydroxyacetonkinase, ist in *S. cerevisiae* jedoch natürlicherweise vorhanden (Molin et al., 2003). Eine Studie von Nguyen und Nevoigt (2009) hat im Übrigen gezeigt, dass die Expression einer heterologen Glycerol-Dehydrogenase aus *Ogataea angusta* (Syn. *Pichia angusta, Hansenula polymorpha*) in *S. cerevisiae* zu deutlich messbarer *in vitro* Enzymaktivität (mit Glycerol als Substrat) geführt hat, während dies bei Überexpression des homologen *GCY1* Gens nicht der Fall war. Jung et al. 2011 haben des Weiteren demonstriert, dass die Expression der Glycerol-Dehydrogenase aus *Ogataea angusta* in *S. cerevisiae* die Glycerol-Nutzung im Vergleich zum Wildtyp-Stamm 1,22-fach verbesserte, d.h. der gentechnisch veränderte Stamm verbrauchte nach 96 h Kultivierung 5,18 g/l von 12,62 g/l Glycerol, während die Kontrolle im Vergleich dazu in der gleichen Zeit nur 4,24 g/l verbrauchte.

Aus der WO 2016/008819 A1 ist eine gentechnisch veränderte Hefe mit verbessertem Glycerol-Katabolismus bekannt. Die Hefezelle wurde dabei dahingehend gentechnisch verändert, dass der Abbau von Glycerol über den G3P-Weg blockiert ist, ein heterologes Glycerolaufnahme-Facilitator-Protein exprimiert wird, und entweder der DHA-Weg etabliert oder verstärkt ist, oder der Glycerinaldehyd-Weg etabliert oder optimiert ist.

Allerdings ist ein fermentativer Abbau von Glycerol zu beispielsweise Bernsteinsäure durch eukaryotische Organismen wie Hefe bislang nicht möglich. Vielmehr müssen hierfür Bakterien eingesetzt werden, obwohl es wünschenswert wäre, auch Hefe zur Fermentation von Glycerol einsetzen zu können.

Aufgabe der vorliegenden Erfindung ist es, Hefe zur fermentativen Nutzung von Glycerol zur Herstellung von C₄-Dicarbonsäuren, z. B. Bernsteinsäure, zu befähigen bzw. die Fähigkeit von Hefe zu verbessern, Glycerol als Substrat zur fermentativen Herstellung von C₄-Dicarbonsäuren zu nutzen. Insbesondere soll durch die Erfindung die Herstellung von C₄-Dicarbonsäuren, z. B. Bernsteinsäure, durch Hefe unter mikroaeroben oder bevorzugt anaeroben Bedingungen ermöglicht werden, um sowohl den Kohlenstoff als auch die Reduktionskraft (in Form von NADH) maximal in die Produktbildung zu lenken.

Gelöst wird die Aufgabe durch eine gentechnisch veränderte Hefezelle der Gattung *Saccharomyces* zur Fermentation von Glycerol, wobei die Hefezelle dahingehend gentechnisch verändert ist, dass
i) der Abbau von Glycerol zu Dihydroxyacetonphosphat über den Glycerol-3-Phosphat-Weg blockiert ist,
ii) ein heterologes Glycerolaufnahme-Facilitator-Protein exprimiert wird,
iii) eine die Oxidation von Glycerol zu Dihydroxyaceton katalysierende heterologe Glyceroldehydrogenase exprimiert oder eine die Oxidation von Glycerol zu Dihydroxyaceton katalysierende native Glyceroldehydrogenase überexprimiert wird,
iv) eine heterologe Dihydroxyacetonkinase exprimiert oder eine native Dihydroxyacetonkinase überexprimiert wird,
v) ein heterologer C4-Dicarbonsäure-Transporter exprimiert wird,
vi) eine heterologe Glyceroldehydratase exprimiert wird,
vii) eine heterologe 1,3-PDO-Oxidoreduktase exprimiert wird,
viii) eine heterologe PEP-Carboxykinase exprimiert oder die native PEP-Carboxykinase überexprimiert wird, und
ix) die Pyruvat-Carboxylase deaktiviert ist.

Die erfindungsgemäß gentechnisch veränderte Hefezelle ist in der Lage, Glycerol als einziges Substrat auf rein fermentativem Weg, d.h. unter anaeroben Bedingungen, oder zumindest unter stark sauerstofflimitierten (mikroaeroben) Bedingungen, vorwiegend zu C₄-Dicarbonsäuren, insbesondere Bernsteinsäure, als Produkt umzusetzen. Die Herstellung von C₄-Dicarbonsäuren, beispielsweise Bernsteinsäure, durch Hefe, insbesondere die in der Biotechnologie verbreitet genutzte Bäckerhefe (*Saccharomyces cerevisiae*), ist gegenüber der Herstellung durch Bakterien vorteilhaft, weil der Prozess beispielsweise bei niedrigem pH erfolgen kann, wodurch auf den Einsatz neutralisierender Verbindungen verzichtet werden kann. Die erfindungsgemäße Hefezelle ist in einer Weise gentechnisch verändert, dass die cytosolische Redoxbilanz ausgeglichen ist.

Die Hefezelle gemäß der vorliegenden Erfindung zeichnet sich im Wesentlichen durch folgende Merkmale aus:
1. Energieunabhängiger Glycerol-Import
2. Glycerol-Abbau über den DHA-Weg unter Bildung von cytosolischem NADH
3. Verbesserter Export gebildeter C₄-Carbonsäuren in das umgebende Medium
4. Stoffwechselweg zur Bildung von 1,3-Propandiol als Redoxsenke für bei der Biomasseproduktion erzeugtes NADH
5. Optimierte cytosolisch reduktive Bildung von C₄-Dicarbonsäuren durch verstärkte direkte Bildung von Oxaloacetat aus Phosphoenolpyrvuvat

Die erfindungsgemäß vorgesehene Kombination von gentechnischen Veränderungen an der *Saccharomyces*-Zelle bewirkt eine effiziente Aufnahme und Kanalisierung von Glycerol durch den nunmehr etablierten oder zumindest verstärkten DHA-Stoffwechselweg und ermöglicht der Zelle die Bildung von C₄-Dicarbonsäuren, insbesondere Bernsteinsäure, unter anaeroben oder zumindest mikroaeroben Bedingungen. Die Integration eines heterologen Glycerol-Facilitators fördert die Aufnahme von Glycerol, ohne dass hierfür Energie benötigt wird. Als heterologer Glycerol-Facilitator kommt beispielsweise *FPS2* aus *Pachysolen tannophilus* oder *FPS1* aus *Cyberlindnera* (*Pichia*) *jadinii* in Frage.

Die Etablierung oder Stärkung des DHA-Weges bei gleichzeitiger Blockade des Glycerol-3-phosphat-Wegs führt dazu, dass in die Zelle aufgenommenes Glycerol über den DHA-Weg abgebaut wird, wobei durch die Einführung einer heterologen Glyceroldehydrogenase oder die Überexpression eines nativen Enzyms mit Glyceroldehydrogenaseaktivität der etwaige Mangel an einer natürlichen Glyceroldehydrogenaseaktivität kompensiert ist. Eine ungenügende Aktivität der natürlichen Dihydroxyacetonkinase dagegen wird durch Überexpression der nativen Dihydroxyacetonkinase oder durch Expression einer heterologen Dihydroxyacetonkinase erhöht. Darüber hinaus ist der L-G3P-Weg nur unter aeroben Bedingungen funktionsfähig, da die daran beteiligte FAD-abhängige Glycerol-3-phosphat-Dehydrogenase auf der Außenseite der inneren Mitochondrienmembran lokalisiert ist (Gancedo et al. 1968; Klingenberg 1970) und Elektronen aus der Oxidation von Glycerol-3-phosphat direkt in die mitochondriale Atmungskette mit Sauerstoff als finalem Elektronenakzeptor leitet. Das ist darüber hinaus auch nachteilig, wenn Reduktionsäquivalente beispielsweise für eine nachfolgende Herstellung reduzierter Verbindungen benötigt werden. Über den DHA-Weg kann Glycerol dagegen sowohl unter aeroben als auch unter anaeroben Bedingungen abgebaut werden.

Bei der erfindungsgemäßen gentechnisch veränderten Hefezelle ist auch der Export von C₄-Dicarbonsäuren, insbesondere Bernsteinsäure, aus der Zelle optimiert. Hierzu weist die Zelle einen heterologen C₄-Dicarbonsäure-Transporter, vorzugsweise einen Succinat-Transporter, beispielsweise aus *Aspergillus niger* (s. Los et al., 2015), auf.

Bei der erfindungsgemäßen Hefezelle ist zudem ein vorzugsweise B 12-unabhängiger Stoffwechselweg zur Bildung von 1,3-Propandiol als Redoxsenke für bei der Biomasseproduktion gebildetes überschüssiges NADH etabliert. Die reduktive Herstellung von Bernsteinsäure aus Glycerol ist redoxneutral. Bei Hefe kommt es bei der Biomassebildung während des Wachstums jedoch zur Bildung eines Überschusses von cytosolischem NADH (Oura, 1977). Insbesondere für die fermentative Herstellung von C₄-Dicarbonsäuren, beispielsweise Bernsteinsäure, aus Glycerol ist daher erfindungsgemäß ein heterologer Stoffwechselweg (1,3-PDO-Weg) zur Bildung von 1,3-Propandiol als Redoxsenke vorgesehen, um überschüssiges cytosolisches NADH zu reoxidieren, das während der Biomassebildung bzw. des Wachstums gebildet wird. Der 1,3-PDO-Weg ist aus Bakterien, insbesondere der Familien *Enterobacteriaceae, Clostridiaceae* und *Lactobacillaceae* bekannt (Biebl et al., 1999, Celinska, 2010). Die Bildung von 1,3-PDO ist jedoch auch für gentechnisch veränderte Hefen beschrieben worden (Celinska and Grajek, 2013; Hong et al., 2011; Ma et al., 2010; Rao et al., 2008). Der erste Schritt vom Glycerol aus wird bei diesem Stoffwechselweg katalysiert durch eine Glycerol-Dehydratase, die Glycerol durch Eliminierung eines Moleküls H₂O zu 3-Hydroxypropionaldehyde (3-HPA) umsetzt. Anschließend wird durch eine NADH-abhängige 1,3-Propandiol-Oxidoreduktase aus 3-HPA 1,3-PDO gebildet. Die Glycerol-Dehydratasen gehören zu zwei verschiedenen Enzymfamilien (Feliks und Ullmann, 2012). Eine Gruppe hängt von dem Cofaktor Vitamin B12 ab, die andere nicht. Im Rahmen der vorliegenden Erfindung ist ein Enzym aus der Gruppe der B12-unabhängigen Glycerol-Dehydratasen bevorzugt. Ein Beispiel für eine B 12-unabhängige Glycerol-Dehydratase ist die Dehydratase aus *Clostridium butyricum* (Raynaud et al., 2003). Da die von *dhaB1* kodierte Dehydratase durch das Substrat Glycerol inaktiviert wird (Mori et al., 1997), ist zu ihrer Reaktivierung ein Reaktivierungsfaktor erforderlich, der von *dhaB2* kodiert wird.

Unter dem Begriff "Glycerol-3-Phosphat-Weg", "Glycerol-3-Phosphat-Stoffwechselweg", "G3P-Weg" oder auch "Phosphorylierungsweg" wird die Umsetzung von Glycerol über L-Glycerol-3-phosphat (L-G3P, G3P) zu Dihydroxacetonphosphat (DHAP) verstanden.

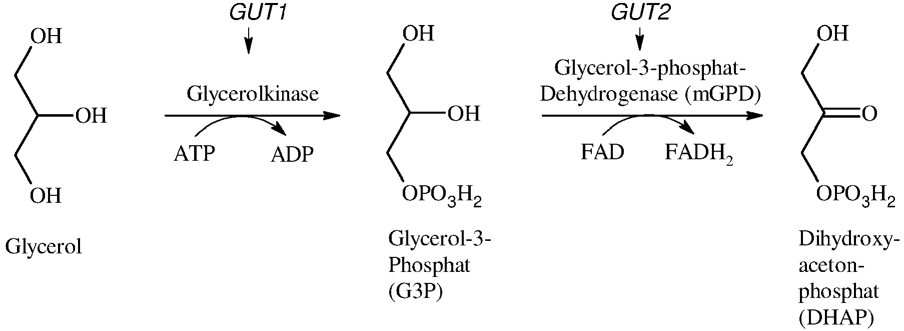

Die Reaktionen werden von der Glycerolkinase und der mitochondrialen (FADabhängigen) Glycerol-3-phosphat-Dehydrogenase (GPD-M, mGPD, EC 1.1.5.3) katalysiert. In der Hefe *Saccharomyces cerevisiae* wird die Glycerolkinase durch das Gen *GUT1,* die Glycerol-3-phosphat-Dehydrogenase durch *GUT2* kodiert.

Unter dem "DHA-Weg", "DHA-Stoffwechselweg" oder "Oxidationsweg" wird die Umsetzung von Glycerol über Dihydroxyaceton (DHA) zu Dihydroxacetonphosphat (DHAP) verstanden.

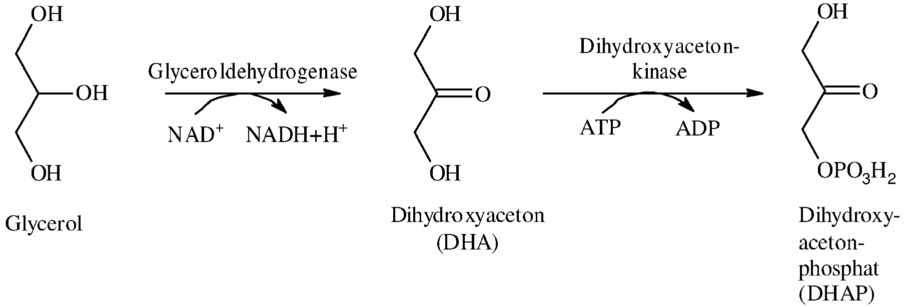

Die Reaktionen werden von der Glyceroldehydrogenase (EC 1.1.1.6) und der Dihydroxyacetonkinase (EC 2.7.1.29) katalysiert.

Unter dem Begriff "Glycerinaldehyd-Weg" wird die Umsetzung von Glycerol über D-Glycerinaldehyd zu D-Glycerinaldehyd-3-Phosphat (GAP) verstanden.

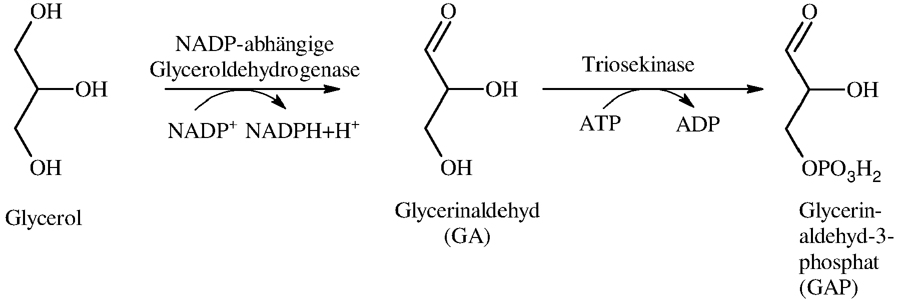

Die Reaktionen werden von einer NADP-abhängigen Glyceroldehydrogenase (NADP-Glyceroldehydrogenase, NADP-GDH, EC 1.1.1.72 bzw. 1.1.1.372) und einer Glycerinaldehyd-Kinase (Triokinase, Triosekinase, EC 2.7.1.28) katalysiert.

Wenn hier von einem "Syntheseweg zur Rückbildung von Glycerol aus Dihydroxyacetonphosphat" gesprochen wird, ist damit die Umsetzung von Dihydroxyacetonphosphat über Glycerol-3-phosphat zu Glycerol gemeint.

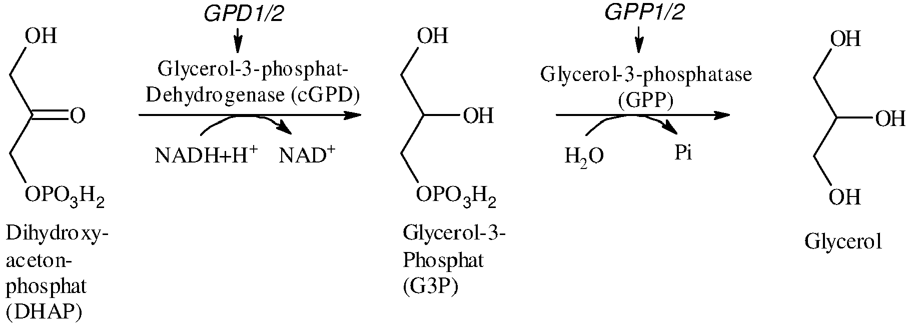

Die Reduktion von DHAP zu Glycerol-3-phosphat wird durch die in zwei Isoformen vorkommende cytosolische NAD⁺-abhängige Glycerol-3-phosphat-Dehydrogenase (GPD-C, cGPD, EC 1.1.1.8) katalysiert. Die Isoformen werden von den Genen *GPD1* bzw. *GPD2* kodiert. Die Dephosphorylierung von Glycerol-3-phosphat zu Glycerol wird von der Glycerol-3-phosphatase (GPP, EC 3.1.3.21) katalysiert, die ebenfalls in zwei Isoformen vorkommt, die von *GPP1* und *GPP2* kodiert werden.

Unter dem Begriff "1,3-Propandiol-Weg" oder "1,3-PDO-Weg" wird die Umsetzung von Glycerol über 3-Hydroxypropionaldehyd (3-HPA) zu 1,3-Propandiol (1,3-PDO, 1,3-PD) verstanden.

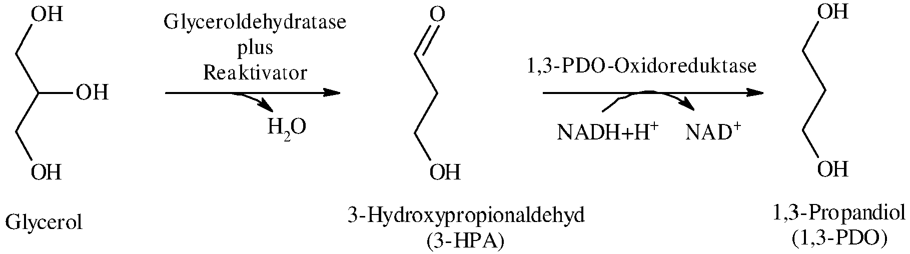

Die Umsetzung von Glycerol zu 3-Hydroxypropionaldehyd (3-Hydroxypropanal, 3-HPA) wird durch Glyceroldehydratase (GDHt, EC 4.2.1.30) katalysiert. Bevorzugt ist im Rahmen der vorliegenden Erfindung eine B 12-unabhängige Glyceroldehydratase aus *Clostridium butyricum,* die durch *dhaB1* kodiert wird. Diese benötigt ein Reaktivatorprotein, das von *dhaB2* kodiert wird. 3-HPA wird durch die, vorzugsweise ebenfalls aus C. *butyricum* stammende, 1,3-Propandiol-Oxidoreduktase (PDOR, EC 1.1.1.202) zu 1,3-PDO reduziert.

Unter einem "B12-unabhängigen 1,3-PDO-Weg" wird die Umsetzung von Glycerol über 3-Hydroxypropionaldehyd zu 1,3-Propandiol mittels B12-unabhängiger Enzyme, insbesondere mittels einer B12-unabhängigen Glyceroldehydratase (EC 4.2.1.30), verstanden.

Der Begriff "heterolog" wird hier in seiner dem Fachmann bekannten Bedeutung verwendet, und bezieht sich auf die fremde Herkunft eines Elements, beispielsweise eines Enzyms oder anderen Proteins. "Fremd" bedeutet, dass das Element so in der Zielzelle nicht vorkommt, und beispielsweise aus einer Zelle oder einem Organismus mit abweichender genetischer Ausstattung, beispielsweise einem Organismus einer anderen Art, stammt.

Der Begriff "homolog" wird hier in Bezug auf ein Enzym oder Protein gegebenenfalls verwendet, um dieses als ein natives, d.h. in der Zielzelle so auch natürlicherweise vorkommendes Enzym oder Protein zu kennzeichnen, im Gegensatz zu einem heterologen Enzym/Protein.

Unter einem "Glycerolaufnahme-Facilitator-Protein" oder kurz "Glycerol-Facilitator" wird ein Transmembranprotein verstanden, das den Transport von Glycerol in eine Zelle, beispielsweise eine *Saccharomyces-Zelle,* mittels erleichterter Diffusion ermöglicht.

Unter "Glyceroldehydrogenase" (kurz GDH) wird hier eine cytosolische Glycerol:NAD⁺-Oxidoreduktase (EC 1.1.1.6) verstanden, welche die Oxidation von Glycerol zu Dihydroxyaceton mittels NAD⁺ als Kofaktor katalysiert. Unter einer "heterologen Glyceroldehydrogenase" wird eine cytosolische Fremd-Glyceroldehydrogenase verstanden, d.h. eine aus einem anderen Organismus, z.B. einer anderen Art, Gattung etc., stammende Glyceroldehydrogenase, die jedoch dieselbe Reaktion katalysiert, gegebenenfalls unter Nutzung eines anderen Kofaktors (insbesondere NADP⁺).

Unter dem Begriff "NADP-Glyceroldehydrogenase" (abgekürzt auch "NADP-GDH") wird hier eine Glycerol:NADP+-Oxidoreductase (EC 1.1.1.72) verstanden, die Glycerol zu D-Glycerinaldehyd oxidiert, wobei NADP+ als Kofaktor fungiert.

Unter dem Begriff "Dihydroxyacetonkinase" oder "DHA-Kinase" wird hier eine ATP:Dihydroxyaceton-Phosphotransferase (EC 2.7.1.29) verstanden, die die Phosphorylierung von Dihydroxaceton mittels ATP katalysiert.

Unter "Pyruvat-Carboxylase" (EC 6.4.1.1) wird hier ein Enzym verstanden, das die Bildung von Oxalacetat durch Addition von Kohlenstoffdioxid an Pyruvat katalysiert. Die katalytische Reaktion verbraucht ATP.

Unter "Phosphoenolpyruvat-Carboxykinase" (EC 4.1.1.49, PEP-Carboxykinase, PEPCK, ATP-PEPCK) wird ein Enzym verstanden, das die Bildung von Oxalacetat aus Phosphoenolpyruvat (PEP) durch Addition von Kohlenstoffdioxid an PEP unter Bildung von ATP katalysiert.

Unter einer "cytosolischen Malatdehydrogenase" (EC 1.1.1.37, MDH) wird ein im Cytoplasma vorkommendes Enzym verstanden, das die Reduktion von Oxalacetat zu Malat mittels NADH katalysiert. Der Begriff schließt auch peroxisomale Malatdehydrogenasen ein.

Unter einer "Fumarase" (EC 4.2.1.2, Fumarat-Hydratase, (S)-Malat-Hydrolyase) wird ein Enzym des Tricarbonsäurezyklus verstanden, das die Bildung von Fumarat aus Malat katalysiert.

Unter einer "Fumaratreduktase" (EC 1.3.1.6, EC 1.3.5.4, EC:1.3.5.1, FRD) wird ein Enzym des Tricarbonsäurezyklus verstanden, das Fumarat zu Succinat reduziert, vorzugsweise eine NADH-abhängige Fumaratreduktase (EC 1.3.1.6).

Unter einem "Malatenzym" (EC 1.1.1.38, EC 1.1.1.39, EC 1.1.1.40) wird ein Enzym verstanden, das Pyruvat zu Malat umsetzt. Das Oxalacetat-decarboxylierende Malatenzym (EC 1.1.1.38, auch Oxalacetat-decarboxylierende Malatdehydrogenase oder NAD-Malatenzym) ist NAD-abhängig und kann auch Oxalacetat decarboxylieren, das decarboxylierende Malatenzym (EC 1.1.1.39, auch decarboxylierende Malatdehydrogenase oder NAD-Malatenzym) ist ebenfalls NAD-abhängig, decarboxyliert Oxalacetat jedoch nicht, das NADP-abhängige Malatenzym (EC 1.1.1.40) decarboxyliert Oxalacetat ebenfalls. Wenn hier der Begriff "Oxalacetat-decarboxylierendes Malatenzym", ggfs. auch "Oxalacetat-decarboxylierende Malatdehydrogenase", verwendet wird, ist damit das Oxalacetat-decarboxylierende Malatenzym EC 1.1.1.38 gemeint.

Unter "Pyruvatkinase" (EC 2.7.1.40) wird ein Enzym verstanden, das Phosphoenolpyruvat unter ATP-Bildung zu Pyruvat umsetzt.

Unter "Oxalacetat-Decarboxylase" wird eine Pyruvat bildende Oxalacetat-Carboxylyase (EC 4.1.1.3) verstanden, ein Enzym, welches die Decarboxylierung von Oxalacetat zu Pyruvat katalysiert.

Unter "C4-Dicarbonsäuren" werden Dicarbonsäuren, d.h. Carbonsäuren mit zwei CarboxyGruppen (-COOH), mit 4 C-Atomen verstanden. Beispielse für C4-Dicarbonsäuren sind Bernsteinsäure (Butandisäure, Succinylsäure, C₄H₆O₄), Fumarsäure (trans-Butendisäure, C₄H₄O₄) und Äpfelsäure (2-Hydroxybutandisäure, C₄H₆O₅). Gegebenenfalls werden hier synonym auch die Begriffe, die überlicherweise die Salze der Dicarbonsäuren bezeichnen, verwendet, d.h. für Bernsteinsäure "Succinat", für Fumarsäure "Fumarat" und für Äpfelsäure "Malat".

Unter einem "C4-Dicarbonsäure-Transporter" wird ein Transmembranprotein verstanden, das den Transport einer C4-Dicarbonsäure wie beispielsweise Äpfelsäure, Fumarsäure oder Bernsteinsäure über eine Zellmembran hinweg, d.h. aus einer Zelle, beispielsweise einer *Saccharomyces*-Zelle, heraus oder in die Zelle hinein ermöglicht. Der Begriff schließt auch Proteine ein, die neben C4-Dicarbonsäuren weitere Moleküle transportieren.

Unter einem "Glyceroldehydratase-Reaktivator", "Glyceroldehydratase-Aktivator" oder einer Glyceroldehydratase-Aktivase (GD-AE) wird hier ein zur Superfamilie der Radikal-SAM-Enzyme gehörendes Enzym verstanden, das eine Glyceroldehydratase (re)aktiviert. Ein Beispiel ist der vom Gen *dhaB2* kodierte Glyceroldehydratase-Reaktivator (DhaB2) aus *Clostridium butyricum* (Raynaud et al. 2003).

Unter "Expression" wird hier die Umsetzung einer genetischen Information in ein Produkt verstanden, beispielsweise die Bildung eines Proteins oder einer Nukleinsäure anhand der genetischen Information. Der Begriff umfasst insbesondere die Biosynthese von Proteinen anhand der genetischen Information einschließlich vorangehender Prozesse wie der Transkription, d.h. der Bildung von mRNA auf Basis einer DNA-Vorlage.

Unter "Überexpression" wird eine Expression eines Gens verstanden, die im Vergleich zu einer Expression desselben Gens stärker ist, d.h. zu mehr Genprodukt führt. In der Regel wird hier von Überexpression gesprochen, wenn ein natives, d.h. ein in der Zelle natürlicherweise vorkommendes, Genprodukt stärker als unter natürlichen Bedingungen exprimiert wird. Eine Überexpression kann beispielsweise durch Einführung zusätzlicher Kopien des für das Genprodukt kodierenden Gens in eine Zelle und/oder die Ersetzung des natürlichen Promotors, unter dessen Kontrolle die Expression des Gens erfolgt, durch einen stärkeren Promotor bewirkt werden.

Der Ausdruck, dass ein heterologes Protein/Enzym exprimiert wird, schließt ein, dass das Protein/Enzym im Vergleich zum durchschnittlichen Expressionsniveau des Proteins/Enzyms in der Herkunftszelle in der Zielzelle unter ansonsten gleichen Bedingungen überexprimiert wird. Der Ausdruck schließt auch ein, dass das Protein/Enzym unter Kontrolle eines beliebigen Promotors exprimiert wird, also auch eines Promotors, der von dem Promotor verschieden sein kann, der in der Herkunftszelle mit dem Protein/Enzym verknüpft ist oder der in der Zielzelle mit einem etwaigen orthologen Protein/Enzym verknüft ist. Dem Fachmann ist bekannt, wie er eine Überexpression eines Proteins/Enzyms bewirken kann. Eine Möglichkeit ist beispielsweise die Einführung zusätzlicher Kopien des für das Protein/Enzym kodierenden Gens in eine Zelle und/oder die Ersetzung des natürlichen Promotors, unter dessen Kontrolle die Expression des Gens erfolgt, durch einen stärkeren Promotor.

Unter dem Begriff der "Blockade" oder des "Blockierens" eines Stoffwechselweges, d.h. einer Kette von Reaktionen mit einer Ausgangsverbindung, mindestens einem Zwischenprodukt und einem Endprodukt (das selbst auch wieder die Ausgangsverbindung für einen anderen Stoffwechselweg sein kann) wird hier verstanden, dass die Bildung zumindest des ersten Zwischenproduktes des Weges unterbunden oder wesentlich vermindert ist. "Blockade" oder "Blockieren" bedeutet hier insbesondere, dass im Vergleich zu einer Hefezelle, bei der der Stoffwechselweg nicht blockiert ist, die Bildung des ersten Zwischenprodukts um mindestens 75 %, vorzugsweise mindestens 80 %, mindestens 85 %, mindestens 90 %, mindestens 95 % oder mindestens 98 % vermindert ist. In Bezug auf den Glycerol-3-Phosphat-Stoffwechselweg wird unter einer Blockade daher verstanden, dass zumindest der Abbau von Glycerol zu Glycerol-3-Phosphat durch die Glycerolkinase blockiert ist. In Bezug auf den Syntheseweg zur Rückbildung von Glycerol aus Dihydroxyacetonphosphat (DHAP) wird unter einer Blockade verstanden, dass zumindest die Reduktion von DHAP zu Glycerol-3-Phosphat durch Glycerol-3-phosphat-Dehydrogenase oder zumindest die Reduktion von Glycerol-3-Phosphat zu Glycerol durch Glycerol-3-phosphatase blockiert ist. Der Begriff schließt auch ein, dass sämtliche Zwischenschritte auf dem Weg zum Endprodukt blockiert sind, beispielweise durch Deletieren oder Mutieren der Gene sämtlicher an dem Weg beteiligter Enzyme, so dass kein funktionsfähiges Genprodukt oder nur ein in seiner Funktion wesentlich eingeschränktes Genprodukt, d.h. Enzym, resultiert. Der Begriff kann auch bedeuten, dass die Bildung von funktionsfähigem Enzym um die oben genannten Prozentzahlen vermindert ist, indem beispielsweise die natürlichen Promotoren durch entsprechend schwächere ausgetauscht werden, oder dass nur eine solche Menge eines in seiner Funktionalität eingeschränkten Enzyms gebildet wird, dass die Bildung des Zwischenproduktes wie oben definiert vermindert ist. Mittel zur Modifizierung eines Stoffwechselweges im Wege des "Metabolic Engineering" sind dem Fachmann gut bekannt (s. z.B. Nevoigt 2008; Mapelli 2014). Beispielsweise kann ein Stoffwechselweg durch Deletieren oder Deaktivieren eines Gens oder eines für die Regulation eines Gens relevanten DNA-Abschnitts, beispielsweise mittels zielgerichteter Mutation, blockiert werden. Eine Modifikation dahingehend, dass ein heterologes Enzym exprimiert wird, kann durch dem Fachmann bekannte gentechnische Verfahren erreicht werden, beispielsweise indem ein geeigneter Expressionsvektor mit einem das Enzym kodierenden Gen (zusammen mit einem geeigneten Promoter und Terminator) in die Zelle eingebracht wird.

Wenn hier der Ausdruck "Deaktivieren" in Zusammenhang mit einem Enzym verwendet wird, bedeutet dies, dass im Vergleich zu einer Wildtypzelle zumindest eine deutlich verminderte Aktivität des entsprechenden Enzyms vorliegt. Insbesondere bedeutet der Ausdruck, dass die Enzymaktivität im Vergleich zu einer Wildtypzelle um mindestens 75 %, vorzugsweise mindestens 80 %, mindestens 85 %, mindestens 90 %, mindestens 95 %, 96 %, 97%, mindestens 98 % oder mindestens 99% vermindert ist. Bevorzugt ist im Wesentlichen keine Enzymaktivität vorhanden. Eine Deaktivierung eines Enzyms kann beispielsweise durch Deletieren oder Mutieren des das Enzym kodierenden Gens erfolgen. Methoden zur Deaktivierung von Enzymen sind dem Fachmann bekannt. In Zusammenhang mit der vorliegenden Erfindung ist der Ausdruck insbesondere so zu verstehen, dass eine von einem Enzym durchgeführte Reaktion im Vergleich zu einer Wildtypzelle in deutlich vermindertem Umfang stattfindet, vorzusweise im Vergleich zu einer Wildtypzelle in einem um mindestens 75 %, vorzugsweise mindestens 80 %, mindestens 85 %, mindestens 90 %, mindestens 95 %, 96 %, 97%, mindestens 98 % oder mindestens 99% verminderten Umfang. Der Begriff "Enzym" umfasst hier auch sämtliche Isoformen des Enzyms.

Wenn hier von einem Enzym mit einer bestimmten Enzymaktivität gesprochen wird, z.B. von einer Dihydroxyaceton-Kinase (DHA-Kinase), wird von dem Begriff auch ein Enzym erfasst, das neben mindestens einer anderen Enzymaktivität zumindest auch die entsprechende Enzymaktivität hat. Unter einer "DHA-Kinase" beispielsweise wird daher auch ein Enzym mit DHA-Kinase-Aktivität verstanden, dass daneben noch mindestens eine andere Enzymaktivität, beispielsweise eine Triosephosphatisomeraseaktivität, besitzt. Ein konkretes Beispiel hierfür ist die 2,3-Butandioldehydrogenase (Bdh1) aus *Saccharomyces cerevisiae,* von der gezeigt wurde, dass sie auch Glyceroldehydrogenaseaktivität aufweist (Gonzalez et al., 2000). Wenn hier von der Überexpression einer nativen Glyceroldehydrogenase gesprochen wird, umfasst dies daher auch beispielsweise die Überexpression der Bdhl oder eines anderen nativen Enzyms mit entsprechender Aktivität.

Der Begriff "Fermentation" oder "fermentativ" bedeutet, dass ATP ausschließlich oder zumindest weit überwiegend nicht durch oxidative Phosphorylierung, d.h. durch Elektronentransportketten, gewonnen wird. Vorzugsweise bedeutet der Begriff, dass ATP ausschließlich anaerob oder unter mikroaeroben, d.h. unter stark sauerstofflimitierten Bedingungen gewonnen wird, vorzugsweise anaerob. Der Anteil von ATP, der durch Fermentation, d.h. nicht unter Beteiligung von Elektronentransportketten gewonnen wird, liegt bevorzugt bei mindestens 80%, besonders bevorzugt bei mindestens 85%, 90%, 95%, 96%, 97%, 98% oder 99%.

Bei der erfindungsgemäßen gentechnisch veränderten Hefezelle ist die cytosolisch reduktive Bildung von C₄-Dicarbonsäuren, insbesondere Bernsteinsäure, optimiert. Bernsteinsäurebildung aus Glucose als einziger Kohlenstoffquelle durch S. *cerevisiae* wurde von mehreren Autoren beschrieben (Raab et al., 2010; Otero et al., 2013; Yan et al., 2014). Der Stoffwechselweg von Glycerol zu Bernsteinsäure ist redoxneutral (2 mol NADH werden gebildet und 2 mol NADH sind erforderlich), solange das Zellwachstum unberücksichtigt bleibt.

Bei der erfindungsgemäßen Hefezelle ist viii) die Phosphoenolpyruvat-Carboxykinase (PEP-Carboxykinase oder PEPCK) überexprimiert oder durch eine heterologe Phosphoenolpyruvat-Carboxykinase ersetzt (s. Zelle et al. (2010)).

Die verstärkte direkte Bildung von Oxalacetat mittels Phosphoenolpyruvat-Carboxykinase ist besonders vorteilhaft, da hierbei netto ein ATP gebildet wird, während dies bei dem Weg über Pyruvatkinase (konvertiert Phosphoenolpyruvat zu Pyruvat) und Pyruvat-Carboxylase nicht der Fall ist.

Bei dieser Ausführungsform der erfindungsgemäßen gentechnisch veränderten Hefezelle ist die cytosolisch reduktive Bildung von C₄-Dicarbonsäuren, insbesondere Bernsteinsäure, weiterhin optimiert, indem ix) die Pyruvat-Carboxylase deaktiviert ist.

Dies ist vorteilhaft, um den Weg der direkten Bildung von Oxalacetat aus Phosphoenolpyruvat mittels Phosphoenolpyruvat-Carboxykinase, bei dem im Gegensatz zum Weg über Pyruvat netto ein ATP gebildet wird, weiter zu stärken bzw. die Bildung von Oxalacetat aus Pyruvat unter ATP-Verbrauch zu unterbinden.

Bevorzugt ist die Hefezelle dahingehend gentechnisch verändert, dass
x) eine heterologe Oxalacetat-Decarboxylase exprimiert oder eine native Oxalacetat-Decarboxylase überexprimiert wird, und/oder
xi) ein heterologes Oxalacetat-decarboxylierendes Malatenzym exprimiert oder ein natives Oxalacetat-decarboxylierendes Malatenzym überexprimiert wird.

Dies ist besonders vorteilhaft, um die Bildung von Pyruvat aus Oxalacetat zu ermöglichen bzw. zu fördern. Die von der Pyruvat-Carboxylase katalysierte Bildung von Oxalacetat aus Pyruvat ist unter physiologischen Bedingungen regelmäßig irreversibel. Sowohl die Oxalacetat-Decarboxylase als auch das Oxalacetat-decarboxylierende Malatenzym (1.1.1.38) katalysieren jedoch die Bildung von Pyruvat aus Oxalacetat, wobei kein ATP gebildet oder verbraucht wird.

Die Expression einer heterologen Oxalacetat-Decarboxylase oder Überexpression einer nativen Oxalacetat-Decarboxylase, und/oder die Expression eines heterologen Oxalacetat-decarboxylierenden Malatenzyms oder die Überexpressoin eines nativen Oxalacetat-decarboxylierenden Malatenzyms ist auch für sich allein genommen vorteilhaft, d.h. wenn die Hefezelle nur in diesem Merkmal, und nicht auch durch Verwirklichung der obigen Merkmal i bis ix, gentechnisch verändert wird. Beispielsweise ist dies nicht nur vorteilhaft für die Optimierung des Glycerol-Stoffwechsels, sondern auch für die Optimierung des Glucose-Stoffwechsels.

In bevorzugten Ausführungsformen ist die erfindungsgemäße Hefezelle zusätzlich dahingehend gentechnisch verändert dass
xii1) eine heterologe cytosolische Malatdehydrogenase exprimiert oder die native cytosolische Malatdehydrogenase überexprimiert wird.

Bei dieser Ausführungsform, die insbesondere für den Fall bevorzugt ist, dass die obigen Merkmale x und xi nicht verwirklicht sind, ist die direkte Bildung von Malat aus Oxalacetat gestärkt. Die Expression einer heterologen cytosolischen Malatdehydrogenase oder Überexpression einer nativen cytosolischen Malatdehydrogenase kann aber auch für den Fall vorgesehen sein, dass eine heterologe Oxalacetat-Decarboxylase exprimiert oder eine native Oxalacetat-Decarboxylase überexprimiert wird, und/oder ein heterologes Oxalacetat-decarboxylierendes Malatenzym exprimiert oder ein natives Oxalacetat-decarboxylierendes Malatenzym überexprimiert wird.

Alternativ kann die Hefezelle jedoch auch dahingehend zusätzlich gentechnisch verändert sein, dass
xii2) die cytosolische Malatdehydrogenase deaktiviert ist.

Bei dieser Ausführungform ist der Weg ausgehend vom Phosphoenolpyruvat über Oxalacetat zum Pyruvat und anschließend zum Malat gestärkt, indem die Umsetzung von Phosphoenolpyruvat direkt zum Pyruvat und/oder die Umsetzung von Oxalacetat direkt zu Malat verhindert oder zumindest stark vermindert ist. Bei dieser Ausführungsform ist vorteilhaft, dass die Reaktion von Phosphoenolpyruvat direkt zu Oxalacetat durch die PEP Carboxylase unter ATP-Gewinnung erfolgt. Bei dieser Ausführungsform ist es bevorzugt, wenn auch eine heterologe Oxalacetat-Decarboxylase exprimiert oder eine native Oxalacetat-Decarboxylase überexprimiert wird, und/oder auch ein heterologes Oxalacetat-decarboxylierendes Malatenzym exprimiert oder ein natives Oxalacetat-decarboxylierendes Malatenzym überexprimiert wird.

Insbesondere bei Ausführungsformen der erfindungsgemäßen Hefezelle, bei denen eine heterologe Oxalacetat-Decarboxylase exprimiert oder eine native Oxalacetat-Decarboxylase überexprimiert wird, und/oder ein heterologes Oxalacetat-decarboxylierendes Malatenzym exprimiert oder ein natives Oxalacetat-decarboxylierendes Malatenzym überexprimiert wird, kann xiii) die Pyruvatkinase deaktiviert sein, um die direkte Bildung von Pyruvat aus Phosphoenolpyruvat zu unterbinden und den Weg über Oxalacetat zu stärken.

Unabhängig davon, ob eine heterologe Oxalacetat-Decarboxylase exprimiert oder eine native Oxalacetat-Decarboxylase überexprimiert wird, und/oder ein heterologes Oxalacetat-decarboxylierende Malatenzym exprimiert oder ein natives Oxalacetat-decarboxylierendes Malatenzym überexprimiert wird, und unabhängig davon, ob die Pyruvatkinase deaktiviert ist oder nicht, kann die Hefezelle auch weiterhin dahingehend gentechnisch verändert sein, dass
xiv) ein heterologes, vorzugsweise NAD-abhängiges, Malatenzym exprimiert oder das native Malatenzym überexprimiert wird.

Für den Fall, dass die Hefezelle bereits dahingehend gentechnisch verändert ist, dass ein heterologes Oxalacetat-decarboxylierende Malatenzym exprimiert oder ein natives Oxalacetat-decarboxylierendes Malatenzym überexprimiert wird, kann auf die Ausbildung dieses Merkmals xiv verzichtet werden. Es kann aber zusätzlich auch die Expression eines weiteren heterologen Malatenzym oder die Überexpression eines weiteren nativen Malatenzyms vorgesehen sein.

Dieses Merkmal xiv ist besonders bevorzugt bei einer Ausführungform der erfindungsgemäßen Hefezelle, bei der eine heterologe Oxalacetat-Decarboxylase exprimiert oder eine native Oxalacetat-Decarboxylase überexprimiert wird, und weiter besonders bevorzugt bei einer Ausführungsform der erfindungsgemäßen Hefezelle, bei der die cytosolische Malatdehydrogenase deaktiviert ist.

Bei der gentechnisch veränderten Hefezelle gemäß der vorliegenden Erfindung handelt es sich vorzugsweise um eine Hefezelle der Art *Saccharomyces cerevisiae.*

In einer bevorzugten Ausführungform der Erfindung ist die Hefezelle darüber hinaus dahingehend gentechnisch verändert, dass zusätzlich
xv) ein heterologer Glyceroldehydratase-Reaktivator exprimiert wird, und/oder
xvi) eine heterologe Fumarase exprimiert wird, und/oder
xvii) eine heterologe Fumaratreduktase exprimiert wird, und/oder
xviii) die Expression des durch *STL1* kodierten natürlichen aktiven Glycerol-Transporters blockiert ist, und/oder
xix) der Syntheseweg zur Rückbildung von Glycerol aus Dihydroxyacetonphosphat blockiert ist und/oder
xx) der Glycerinaldehyd-Weg blockiert ist.

Die Merkmale xv) bis xx) können einzeln oder in beliebiger Kombination oder auch alle zusammen mit den Merkmalen i) bis xiv) verwirklicht sein.

Die Expression eines heterologen Glyceroldehydratase-Reaktivators ist bevorzugt für den Fall, dass eine heterologe Glyceroldehydratase in der Hefezelle exprimiert wird, die reaktiviert werden muss. Für den bevorzugten Fall, dass die B 12-unabhängige Glyceroldehydratase DhaB1 aus *Clostridium butyricum* eingesetzt wird, wird vorzugsweise der Glyceroldehydratase-Reaktivator DhaB2 aus *Clostridium butyricum* eingesetzt.

Die cytosolische Expression einer heterologen Fumarase und/oder die Expression einer heterologen Fumaratreduktase sind bevorzugt, wenn das gewünschte Endprodukt Bernsteinsäure (Succinat) ist. Die heterologen Enzymvarianten weisen bevorzugt eine höhere Umsatzrate auf als die nativen Varianten. Als heterologe Fumarase kommt beispielsweise die durch *fumC* kodierte Fumarase aus *E. coli* in Frage (s. Yan et al. 2014). Als heterologe Fumaratreduktase kommt beispielsweise FRDml aus *Trypanosoma brucei* in Betracht (s. US 20120165569 A1). Es kommen aber auch beispielsweise Fumarasen aus *Rhizopus oryzae* (*fumR*) oder *E. coli* (*fumB*) in Frage.

Durch die Blockade der Expression des natürlichen aktiven durch *STL1* kodierten Transporters wird ein unnötiger Verbrauch von ATP vermieden, insbesondere unter Bedingungen, bei denen die Zellen möglicherweise nicht genügend ATP liefern können, beispielsweise unter Sauerstofflimitierung. Die Expression des heterologen Glycerol-Facilitators ermöglicht dennoch die Aufnahme von Glycerol in die Hefezelle. Die Herstellung einer *STL1*-Deletionsmutante ist beispielsweise in Liu et al. 2013 und Swinnen et al. 2013 beschrieben.

Die Blockade des Synthesewegs zur Rückbildung von Glycerol aus Dihydroxyacetonphosphat verhindert zusätzlich, dass über den DHA-Weg gebildetes Dihydroxyacetonphosphat wieder zu Glycerol-3-Phosphat oder gar Glycerol umgesetzt wird, wodurch insbesondere ein unnötiger Abfluss von Reduktionsäquivalenten vermieden werden kann. Die Blockade kann beispielsweise durch Deletion der Gene (*GPD1*/*2* und/oder *GPP1*/*2*) erreicht werden, welche für die die Rückbildung katalysierenden Enzyme kodieren. Deletionen von *GPD1* bzw. *GPD2* sind in Hubmann et al. (2011) beschrieben. Die Deletion von *GPP1* bzw. *GPP2* ist in Påhlmann et al. (2001) beschrieben.

Alternativ können die natürlichen Promotoren durch sehr viel schwächere ausgetauscht werden, wie es für *GPD1* bzw. *GPD2* bereits beschrieben wurde (Hubmann et al., 2011).

In einer bevorzugten Ausführungsform der vorliegenden Erfindung ist die Hefezelle dahingehend gentechnisch verändert, dass der Abbau von Glycerol zu Dihydroxyacetonphosphat über den Glycerol-3-Phosphat-Weg dadurch blockiert ist, dass die Expression der Glycerolkinase und/oder der mitochondrialen Glycerol-3-Phosphat-Dehydrogenase (mGDP) blockiert ist. Bevorzugt ist, dass sowohl die Expression der Glycerolkinase als auch der mitochondrialen Glycerol-3-Phosphat-Dehydrogenase blockiert ist. Dies kann mit verschiedenen Methoden erreicht werden, die dem Fachmann bekannt sind, beispielsweise durch Deletion der für die Enzyme kodierenden Gene (s. z.B. Swinnen et al. 2013).

Bei dem heterologen Glycerolaufnahme-Facilitator-Protein kann es sich beispielsweise um Fps2 aus *Pachysolen tannophilus* (PtFPS2: JQ481632.1 GL394996127, AFN43531.1 GL394996128; s. Liu et al. 2013) handeln. Bei der heterologen Glyceroldehydrogenase handelt es sich bevorzugt um eine Glyceroldehydrogenase aus *Ogataea angusta* (s. Jung et al. 2011, GenBank: AB185335.1 GI:50582511, BAD32688.1 GI:50582512). Anstelle der Einführung einer heterologen Glyceroldehydrogenase kann auch die in *Saccharomyces cerevisiae* natürlicherweise vorkommende und Glyceroldehydrogenaseaktivität aufweisende Bdhl überexprimiert werden. Als überzuexprimierende Malatdehydrogenase kommt beispielsweise eine peroxisomale MDH3 (YDL078C, Yan et al. 2014) in Frage. Heterologe DHA-Kinasen stammen vorzugsweise aus Organismen, die nachweislich natürlicherweise den DHA-Weg benutzen, z.B. Dakl aus *Schizosaccharomyces pombe* (s. Itoh et al., 1999, UniProtKB/Swiss-Prot O13902, Ver 98, 1106.2014.). Als heterologe 1,3-PDO-Oxidoreduktase ist die von *dhaT* kodierte 1,3-PDO-Oxidoreduktase aus *Clostridium butyricum* (Raynaud et al., 2003) bevorzugt.

In einer weiteren bevorzugten Ausführungsform ist die Hefe zusätzlich dahingehend gentechnisch verändert, dass die Ethanolbildung unterbunden oder stark reduziert ist. Das kann beispielsweise durch Deletion aller Isogene, die für die Pyruvat-Decarboxylase codieren, und anschließendem "Evolutionary Engineering" erfolgen (van Maris et al., 2004). Letzteres ist nötig, um die Auxotrophie einer Hefe ohne Pyruvatdecarboxylase für cytosolisches Acetyl-Co zu überwinden.

In einem weiteren Aspekt betrifft die vorliegende Erfindung auch ein Verfahren zur Herstellung eines Fermentationsproduktes aus Glycerol, wobei eine erfindungsgemäß gentechnisch veränderte Hefezelle nach dem oben beschriebenen Aspekt in einem glycerolhaltigen Kulturmedium unter geeigneten Fermentationsbedingungen, vorzugsweise unter anaeroben oder mikroaeroben Bedingungen, kultiviert und das Fermentationsprodukt von dem Kulturmedium abgetrennt wird. Bevorzugtes Fermentationsprodukt ist eine C4-Dicarbonsäure, besonders bevorzugt Bernsteinsäure (Succinat).

Die Erfindung wird im Folgenden anhand der beigefügten Zeichnung beispielhaft näher erläutert.

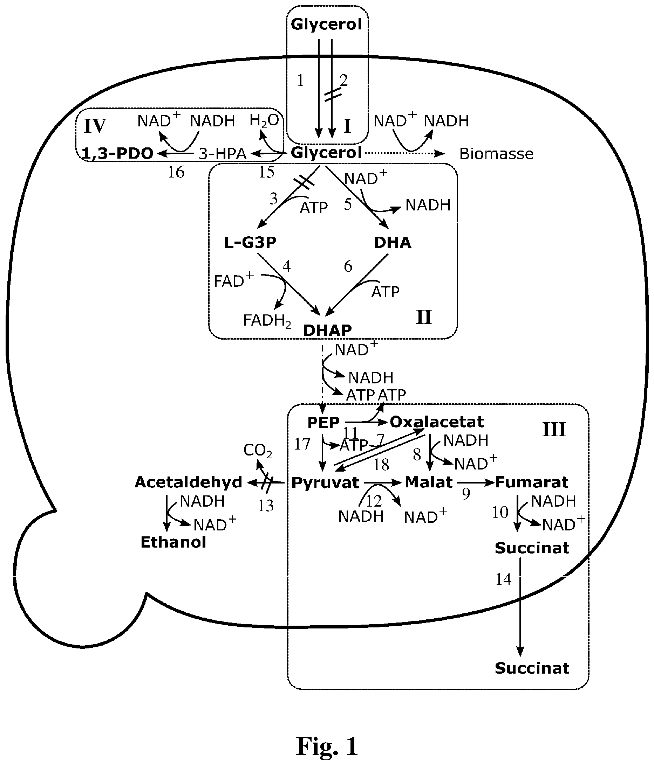

Fig. 1 Vereinfachte schematische Darstellung von an einer Ausführungsform einer erfindungsgemäßen gentechnisch veränderten Hefezelle vorgenommenen Modifikationen für einen fermentativen Abbau von Glycerol zu C4-Dicarbonsäuren. 1 Glycerol-Facilitator, 2 Glycerol-Symporter, 3 Glycerolkinase, 4 G3P-Dehydrogenase, 5 Glyceroldehydrogenase, 6 Dihydroxyacetonkinase, 7 Pyruvat-Carboxylase, 8 cytosolische Malatdehydrogenase, 9 Fumarase, 10 Fumaratreduktase, 11 Phosphoenolpyruvat-Carboxykinase (PEPCK), 12 Malatenzym, 13 Pyruvat-Decarboxylase, 14 C₄-Dicarbonsäure-Transporter, 15 Glycerol-Dehydratase, 16 1,3-Propandiol-Oxidoreduktase, 17 Pyruvatkinase, 18 Oxalacetat-Decarboxylase.

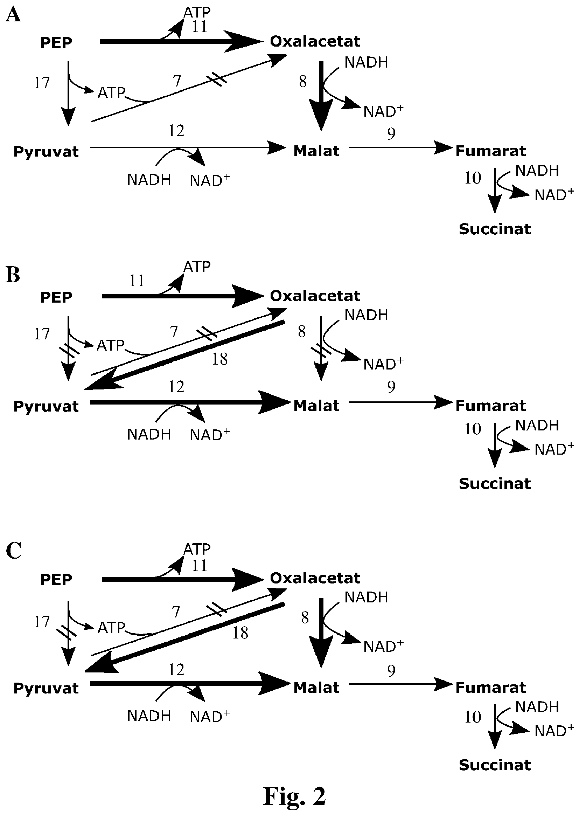

Fig. 2 Schematische Darstellung von Aspekten von an beispielhaften Ausführungsformen einer erfindungsgemäßen gentechnisch veränderten Hefezelle vorgenommenen Modifikationen.

Fig. 1 zeigt schematisch den Abbau von Glycerol zu C4-Dicarbonsäuren bei einer Ausführungsform einer erfindungsgemäßen gentechnisch veränderten Hefezelle. Die Änderungen betreffen im Wesentlichen vier Stoffwechselwege (I-IV, in der Figur umrahmt dargestellt).

### I. Energieunabhängiger Glycerol-Import

Die Integration eines heterologen Glycerol-Facilitators 1, z.B. Fps2 aus *Pachysolen tannophilus,* fördert die Aufnahme von Glycerol, ohne dass hierfür Energie benötigt wird. Vorzugsweise ist gleichzeitig der native energieabhängige Glycerol-Symporter 2 blockiert (Fig. 1, I).

### II. Glycerol-Abbau über den DHA-Weg unter Bildung von cytosolischem NADH

Darüber hinaus ist der DHA-Weg über Glyceroldehydrogenase 5 und Dihydroxyacetonkinase 6 etabliert oder gestärkt, während der Glycerol-3-phosphat-Weg über Glycerolkinase 3 und G3P-Dehydrogenase 4 blockiert ist (Fig. 1, II). Der Glycerol-3-Phosphat-Weg ist beispielsweise durch Deletion der Gene für Glycerolkinase 3 (*GUT1*) und/oder die mitochondriale Glycerol-3-Phosphat-Dehydrogenase (*GUT2*) blockiert. Vorzugsweise ist auch die Rückreaktion über die NAD-abhängige Glycerol-3-Phosphat-Dehydrogenase und die Glycerol-3-Phosphatase durch Deletion der hierfür kodierenden Gene (*GPD1, GPD2, GPP1, GPP2*) blockiert. In die Zelle über den Glycerol-Facilitator transportiertes Glycerol wird über den DHA-Weg über Dihydroxyaceton (DHA) zu Dihydroxyacetonphosphat (DHAP) umgesetzt. Beispielsweise wird hierzu in die Hefezelle eine heterologe Glyceroldehydrogenase, z.B. aus *Ogataea angusta,* exprimiert.

### III. C₄-Dicarbonsäurebildung und Export gebildeter C₄-Dicarbonsäuren in das umgebende Medium

Bei der erfindungsgemäßen gentechnisch veränderten Hefezelle ist darüber hinaus auch die cytosolisch reduktive Bildung und/oder die Ausscheidung von C₄-Dicarbonsäuren, insbesondere Bernsteinsäure (Succinat), optimiert (Fig. 1, III, s. auch Fig. 2). Hierzu ist die Pyruvat-Carboxylase 7 blockiert und die Phosphoenolpyruvat-Carboxykinase (PEPCK) 11 überexprimiert oder durch eine heterologe Phosphoenolpyruvat-Carboxykinase ersetzt. Eine Oxalacetat-Decarboxylase 18 kann exprimiert sein, die Oxalacetat zu Pyruvat umsetzt. Das Malatenzym 12 kann überexprimiert oder durch ein heterologes Malatenzym ersetzt sein. Die cytosolische Malatdehydrogenase 8 (konvertiert Oxalacetat zu Malat) kann überexprimiert oder eine heterologe cytosolische Malatdehydrogenase exprimiert sein. In einigen Ausführungsformen kann die cytosolische Malatdehydrogenase auch deaktiviert sein. In bevorzugten Ausführungsformen kann zusätzlich die Fumarase 9 durch eine heterologe Fumarase ersetzt sein, und/oder kann die Fumaratreduktase 10 durch eine heterologe Fumaratreduktase ersetzt sein. Für einen verbesserten Export von gebildeten C₄-Dicarbonsäuren, z.B. Succinat, aus der Zelle weist die Zelle darüber hinaus einen heterologen C₄-Dicarbonsäure-Transporter 14, vorzugsweise einen Succinat-Transporter, beispielsweise aus *Aspergillus niger,* auf.

### IV. Stoffwechselweg zur Bildung von 1,3-Propandiol als Redoxsenke für bei der Biomasseproduktion erzeugtes NADH

Bei der erfindungsgemäßen Hefezelle ist zudem ein vorzugsweise B 12-unabhängiger Stoffwechselweg zur Bildung von 1,3-Propandiol (1,3-PDO) als Redoxsenke für bei der Biomasseproduktion gebildetes überschüssiges NADH etabliert (Fig. 1, IV). Hierzu sind eine heterologe vorzugsweise B 12-unabhängige Glycerol-Dehydratase 15 und eine heterologe 1,3-Propandiol-Oxidoreduktase 16 in die Zelle eingeführt, die Glycerol über 3-Hydroxypropionaldehyde (3-HPA) zu 1,3-PDO umsetzen. Gegebenenfalls ist darüber hinaus noch ein die heterologe Glycerol-Dehydratase 15 reaktivierender Glycerol-Dehydratase-Reaktivator eingeführt.

Gegebenenfalls kann beispielsweise die Pyruvat-Decarboxylase 13 blockiert sein, um eine Ethanolbildung zu verhindern.

In Figur 2 sind schematisch beispielhafte Modifikationen dargestellt, die insbesondere die C₄-Carbonsäurebildung (s. Fig. 1, III) betreffen. Fett gedruckte Pfeile heben dabei Stoffwechselwege hervor, die durch entsprechende Modifikationen der Hefezelle ausgeführt werden sollen, wobei hier die für die Bildung von Bernsteinsäure (Succinat) aus Malat bevorzugte zusätzliche Modifikation durch Ersetzung der Fumarase 9 durch eine heterologe Fumarase, und/oder durch Ersetzung der Fumaratreduktase 10 durch eine heterologe Fumaratreduktase nicht berücksichtigt sind.

In Fig. 2A sind Modifikationen bei einer Ausführungsform einer gentechnisch veränderten Hefezelle dargestellt, wobei die PEP-Carboxykinase 11 überexprimiert oder durch eine heterologe PEPCK ersetzt ist. Die cytosolische Malatdehydrogenase 8 kann bevorzugt zusätzlich oder alternativ, bevorzugt zusätzlich, überexprimiert oder durch eine heterologe Malatdehydrogenase ersetzt sein. Die Pyruvat-Carboxylase 7 ist deaktiviert. Bei dieser Ausführungsform soll die Umsetzung von PEP vorzugsweise über Oxalacetat und Malat gelenkt werden, ohne Pyruvat wesentlich zu involvieren.

In Fig. 2B sind Modifikationen bei einer weiteren Ausführungsform einer gentechnisch veränderten Hefezelle dargestellt, wobei die PEP-Carboxykinase 11 überexprimiert oder durch eine heterologe PEPCK ersetzt ist. Darüber hinaus ist die Pyruvat-Carboxylase 7 deaktiviert. Darüber hinaus ist vorzugsweise eine heterologe Oxalacetat-Decarboxylase 18 vorgesehen, die Oxalacetat zu Pyruvat umsetzt. Zusätzlich ist hier bevorzugt auch das Malatenzym 12 überexprimiert oder durch ein heterologes Malatenzym ersetzt. Die Überexpression eines Oxalacetat-decarboxylierenden Malatenzyms 12 oder die Einführung eines heterologen Oxalacetat-decarboxylierenden Malatenzyms 12 kann gegebenenfalls die Oxalacetat-Decarboxylase 18 ersetzen. Bevorzugt sind die Pyruvatkinase 17 und/oder die cytosolische Malatdehydrogenase 8, vorzugsweise beide, deaktiviert. Bei dieser Ausführungsform wird der von PEP ausgehende Stoffwechselweg zumindest vorwiegend über Oxalacetat, Pyruvat und Malat gelenkt.

In Fig. 2C sind Modifikationen bei einer weiteren Ausführungsform einer gentechnisch veränderten Hefezelle dargestellt, wobei die PEP-Carboxykinase 11 überexprimiert oder durch eine heterologe PEPCK ersetzt ist, und die Puryvat-Carboxylase 7 deaktiviert ist. Wie bei der in Fig. 2B dargestellten Ausführungsform ist auch hier bevorzugt eine heterologe Oxalacetat-Decarboxylase 18 vorgesehen.

Darüber hinaus ist hier bevorzugt auch die cytosolische Malatdehydrogenase 8 überexprimiert oder durch eine heterologe Malatdehydrogenase ersetzt. Die Pyruvatkinase 17 ist vorzugsweise deaktiviert. Bei dieser Ausführungsform führt der von PEP ausgehende Stoffwechselweg über Oxalacetat zum Pyruvat und Malat, oder direkt von Oxalacetat zu Malat. Das Malatenyzm 12 kann hier gegebenenfalls deaktiviert sein.

Weitere Ausführungsformen können durch entsprechende Kombinationen der erfindungsgemäß vorgesehenen Merkmale vom Fachmann hergestellt werden.

### Referenzen

Biebl, H., Menzel, K., Zeng, A.-P. and W.-D. Deckwer. 1999. Microbial production of 1,3-propanediol. Appl Microbiol Biotechnol 52:289-297.
Borodina, I., Nielsen, Advances in metabolic engineering of yeast Saccharomyces cerevisiae for production of chemicals. J., Biotechnol J. 2014 May;9(5):609-620. doi: 10.1002/biot.201300445.
Celinska, E. 2010. Debottlenecking the 1,3-propanediol pathway by metabolic engineering. Biotech Adv 28:519-530.
Celinska, E. and W. Grajek. 2013. A novel multigene expression construct for modification of glycerol metabolism in Yarrowia lipolytica. Microbial Cell Factories 12:102.
Fakas, S., Makri, A., Bellou, S., Aggelis, G. 2009. Pathways to aerobic glycerol catabolism and their regulation. in: Microbial conversions of raw glycerol, (Ed.) G. Aggelis, Nova Science Publishers, Inc. New York, pp. 9-18.
Feliks, M. and Ullmann, G.M. 2012. Glycerol Dehydratation by the B12-Independent Enzyme May Not Involve the Migration of a Hydroxyl Group: A Computational Study. J Phys Chem B 116:7076-7087.
Ferreira, C., van Voorst, F., Martins, A., Neves, L., Oliveira, R., Kielland-Brandt, M.C., Lucas, C., Brandt, A. 2005. A member of the sugar transporter family, Stllp is the glycerol/H+ symporter in Saccharomyces cerevisiae. Mol Biol Cell, 16(4), 2068-76. Gancedo, C., Gancedo, J.M., Sols, A. 1968. Glycerol metabolism in yeasts. Pathways of utilization and production. Eur J Biochem, 5(2), 165-72.
Gonzalez, E., Fernandez, M.R., Larroy, C., Sola, L., Pericas, M.A., Pares, X., Biosca, J.A. 2000. Characterization of a (2R,3R)-2,3-butanediol dehydrogenase as the Saccharomyces cerevisiae YAL060W gene product. Disruption and induction of the gene. J Biol Chem, 275(46), 35876-85.
Hong, K.K., Nielsen, J. 2012. Metabolic engineering of Saccharomyces cerevisiae: a key cell factory platform for future biorefineries. Cell Mol Life Sci, 69(16), 2671-90.
Hong, W.-K., Kim, C.-H., Heo, S.-Y., et al. 2011. 1,3-Propandiol production by engineered Hansenula polymorpha expressing dha genes from Klebsiella pneumoniae. Bioprocess Biosyst Eng 34:231-236.
Hubmann, G, Guillouet, S., Nevoigt, E. 2011. Gpd1 and Gpd2 Fine-Tuning for Sustainable Reduction of Glycerol Formation in Saccharomyces cerevisiae. Appl. Env. Microbiol. 77, 5857-5867.
Itoh N, Tujibata Y, Liu JQ 1999. Cloning and overexpression in Escherichia coli of the gene encoding dihydroxyacetone kinase isoenzyme I from Schizosaccharomyces pombe, and its application to dihydroxyacetone phosphate production. Appl Microbiol Biotechnol. 51(2):193-200.
Izawa, S., Sato, M., Yokoigawa, K., Inoue, Y. 2004. Intracellular glycerol influences resistance to freeze stress in Saccharomyces cerevisiae: analysis of a quadruple mutant in glycerol dehydrogenase genes and glycerol-enriched cells. Appl Microbiol Biotechnol, 66(1), 108-14.
Jung, J.Y., Yun, H.S., Lee, J., Oh, M.K. 2011. Production of 1,2-propanediol from glycerol in Saccharomyces cerevisiae. J Microbiol Biotechnol, 21(8), 846-53.
Klingenberg, M. 1970. Localization of the glycerol-phosphate dehydrogenase in the outer phase of the mitochondrial inner membrane. Eur J Biochem, 13(2), 247-52.
Lin, E.C. 1976. Glycerol dissimilation and its regulation in bacteria. Annu Rev Microbiol, 30, 535-78.
Liu, X., Mortensen, U.H., Workman, M. 2013. Expression and functional studies of genes involved in transport and metabolism of glycerol in Pachysolen tannophilus. Microb Cell Fact, 12, 27.
Los, A., den Dulk. A., Verwaal, R., et al. September 2015. Metabolic engineering of yeast for commercial production of succinic acid. Poster presented at the 32th International Specialized Symposium on Yeast, Perugia, Italien.
Luyten, K., Albertyn, J., Skibbe, F., Prior, B.A., Ramos, J., Thevelein, J.M., and Hohmann, S. (1995) Fpsl, a yeast member of the MIP-family of channel proteins, is a facilita- tor for glycerol uptake and efflux and it is inactive under osmotic stress. EMBO J 14: 1360-1371.
Ma, Z., Rao, Z., Xu, L., et al. 2010. Expression of dha Operon Required for 1,3-PD Formation in Escherichia coli and Saccharomyces cerevisiae. Curr Microbiol 60:191-198.
McKinlay, J.B., Vieille, C. and J.G. Zeikus. 2007. Prospects for a bio-based succinate industry. Appl Microbiol Biot 76:727 - 740.
Merico, A., Ragni, E., Galafassi, S., Popolo, L., Compagno, C. 2011. Generation of an evolved Saccharomyces cerevisiae strain with a high freeze tolerance and an improved ability to grow on glycerol. J Ind Microbiol Biotechnol, 38(8), 1037-44.
Molin, M., Norbeck, J., Blomberg, A. 2003. Dihydroxyacetone kinases in Saccharomyces cerevisiae are involved in detoxification of dihydroxyacetone. J Biol Chem, 278(3), 1415-23.
Mori, K., Tobimatsu, T., Hara, T. and Toraya, T. 1997. Characterization, sequencing, and expression of the genes encoding a reactivating factor for glycerol-inactivated adenosylcobalamin-dependent diol dehydratase. J Biol Chem 272(51):32034 -32041.
Neves, L., Lages, F., Lucas, C. 2004. New insights on glycerol transport in Saccharomyces cerevisiae. FEBS Lett, 565(1-3), 160-2.
Nevoigt, E. 2008. Progress in metabolic engineering of Saccharomyces cerevisiae. Microbiol Mol Biol Rev, 72(3), 379-412.
Nevoigt, E., Kohnke, J., Fischer, CR., Alper, H., Stahl, U., Stephanopoulos, G. 2006. Engineering of promoter replacement cassettes for fine-tuning of gene expression in Saccharomyces cerevisiae. Appl. Environ. Microbiol. 72: 5266-5273
Nguyen, H.T., Nevoigt, E. 2009. Engineering of Saccharomyces cerevisiae for the production of dihydroxyacetone (DHA) from sugars: a proof of concept. Metab Eng, 11(6), 335-46.
Norbeck, J., Blomberg, A. 1997. Metabolic and regulatory changes associated with growth of Saccharomyces cerevisiae in 1.4 M NaCl. Evidence for osmotic induction of glycerol dissimilation via the dihydroxyacetone pathway. J Biol Chem, 272(9), 5544-54.
Otero, J. M., Cimini, D., Patil, K. R., et al. 2013. Industrial Systems Biology of Saccharomyces cerevisiae Enables Novel Succinic Acid Cell Factory. PLoS ONE 8(1):e54144.
Oura, E. 1977. Reaction products of yeast fermentations. Process Biochem 12:19-21. Raab, A.M., Gebhardt, G., Bolotina, N., et al. 2010. Metabolic engineering of Saccharomyces cerevisiae for the biotechnological production of succinic acid. Metabolic Engineering 12(6):518-25.
Rao, Z., Ma, Z., Shen, W., et al. 2008. Engineered Saccharomyces cerevisiae that produces 1,3-propanediol from D-glucose. J Appl Microbiol 105:1768-1776.
Raynaud, C., Sarcabal, P., Meynial-Salles, I., et al. 2003. Molecular characterization of the 1,3-propanediol (1,3-PD) operon of Clostridium butyricum. PNAS 100(9):5010-5015.
Sprague, G.F., Cronan, J.E. 1977. Isolation and characterization of Saccharomyces cerevisiae mutants defective in glycerol catabolism. J Bacteriol, 129(3), 1335-42.
Swinnen, S., Klein, M., Carrillo, M., McInnes, J., Nguyen, H.T., Nevoigt, E. 2013. Reevaluation of glycerol utilization in Saccharomyces cerevisiae: characterization of an isolate that grows on glycerol without supporting supplements. Biotechnol Biofuels, 6(1), 157.
Tamás, M.J., Luyten, K., Sutherland, F.C., Hernandez, A., Albertyn, J., Valadi, H., Li, H., Prior, B.A., Kilian, S.G., Ramos, J., Gustafsson, L., Thevelein, J.M., Hohmann, S., 1999. Fpslp controls the accumulation and release of the compatible solute glycerol in yeast osmoregulation. Mol. Microbiol. 31, 1087-1104.
Van Maris AJ, Geertman JM, Vermeulen A, Groothuizen MK, Winkler AA, Piper MD, van Dijken JP, Pronk JT., 2004, Directed evolution of pyruvate decarboxylase-negative Saccharomyces cerevisiae, yielding a C2-independent, glucose-tolerant, and pyruvatehyperproducing yeast. Appl Environ Microbiol. 2004 Jan;70(1): 159-66.
Yan, D., Wang, C., Zhou, J., et al. (2014) Construction of reductive pathway in Saccharomyces cerevisiae for effective succinic acid fermentation at low pH value. Bioresource Technology 156: 232-239.
Zelle, R. M., Trueheart, J., Harrison, J. C., et al. 2010. Phosphoenolpyruvate carboxykinase as the sole anaplerotic enzyme in Saccharomyces cerevisiae. Applied and Environmental Microbiology 76:5383-5389.

## Patentansprüche

1. Gentechnisch veränderte Hefezelle der Gattung *Saccharomyces* zur Fermentation von Glycerol, wobei die Hefezelle dahingehend gentechnisch verändert ist, dass
i) der Abbau von Glycerol zu Dihydroxyacetonphosphat über den Glycerol-3-Phosphat-Weg blockiert ist,
ii) ein heterologes Glycerolaufnahme-Facilitator-Protein exprimiert wird,
iii) eine die Oxidation von Glycerol zu Dihydroxyaceton katalysierende heterologe Glyceroldehydrogenase exprimiert oder eine die Oxidation von Glycerol zu Dihydroxyaceton katalysierende native Glyceroldehydrogenase überexprimiert wird,
iv) eine heterologe Dihydroxyacetonkinase exprimiert oder eine native Dihydroxyacetonkinase überexprimiert wird,
v) ein heterologer C4-Dicarbonsäure-Transporter exprimiert wird,
vi) eine heterologe Glyceroldehydratase exprimiert wird,
vii) eine heterologe 1,3-PDO-Oxidoreduktase exprimiert wird,
viii) eine heterologe PEP-Carboxykinase exprimiert oder die native PEP-Carboxykinase überexprimiert wird, und
ix) die Pyruvat-Carboxylase deaktiviert ist.

2. Gentechnisch veränderte Hefezelle nach Anspruch 1, wobei die Hefezelle zusätzlich dahingehend gentechnisch verändert ist, dass
x) eine heterologe Oxalacetat-Decarboxylase exprimiert oder eine native Oxalacetat-Decarboxylase überexprimiert wird, und/oder
xi) ein heterologes Oxalacetat-decarboxylierendes Malatenzym exprimiert oder ein natives Oxalacetat-decarboxylierendes Malatenzym überexprimiert wird.

3. Gentechnisch veränderte Hefezelle nach Anspruch 2, wobei die Hefezelle zusätzlich dahingehend gentechnisch verändert ist, dass
xii1) eine heterologe cytosolische Malatdehydrogenase exprimiert oder die native cytosolische Malatdehydrogenase überexprimiert wird, oder
xii2) die cytosolische Malatdehydrogenase deaktiviert ist, oder.

4. Gentechnisch veränderte Hefezelle nach einem der Ansprüch 2 oder 3, wobei die Hefezelle zusätzlich dahingehend gentechnisch verändert ist, dass xiii) die Pyruvatkinase deaktiviert ist.

5. Gentechnisch veränderte Hefezelle nach einem der vorhergehenden Ansprüche, wobei die Hefezelle weiterhin dahingehend gentechnisch verändert ist, dass xiv) ein heterologes Malatenzym exprimiert oder das native Malatenzym überexprimiert wird.

6. Gentechnisch veränderte Hefezelle nach einem der vorhergehenden Ansprüche, wobei die Hefezelle eine Hefezelle der Art *Saccharomyces cerevisiae* ist.

7. Gentechnisch veränderte Hefezelle nach einem der vorhergehenden Ansprüche, wobei zusätzlich
xv) ein heterologer Glyceroldehydratase-Reaktivator exprimiert wird, und/oder
xvi) eine heterologe Fumarase exprimiert wird, und/oder
xvii) eine heterologe Fumaratreduktase exprimiert oder die native Fumaratreduktase überexprimiert wird, und/oder
xviii) die Expression des durch *STL1* kodierten natürlichen aktiven Glycerol-Transporters blockiert ist, und/oder
xix) der Syntheseweg zur Rückbildung von Glycerol aus Dihydroxyacetonphosphat blockiert ist und/oder
xx) der Glycerinaldehyd-Weg blockiert ist.

8. Gentechnisch veränderte Hefezelle nach einem der vorhergehenden Ansprüche, wobei der Abbau von Glycerol zu Dihydroxyacetonphosphat über den Glycerol-3-Phosphat-Weg dadurch blockiert ist, dass die Expression der Glycerolkinase und/oder der mitochondrialen Glycerol-3-Phosphat-Dehydrogenase blockiert ist.

9. Gentechnisch veränderte Hefezelle nach einem der vorhergehenden Ansprüche, wobei das heterologe Glycerolaufnahme-Facilitator-Protein Fps2 aus *Pachysolen tannophilus* ist, die heterologe Glyceroldehydrogenase eine Glyceroldehydrogenase aus *Ogataea angusta* ist, der heterologe C4-Dicarbonsäure-Transporter ein Succinat-Transporter aus *Aspergillus niger* ist und/oder die heterologe 1,3-PDO-Oxidoreduktase aus *Clostridium butyricum* ist.

10. Verfahren zur Herstellung eines Fermentationsproduktes aus Glycerol, wobei eine gentechnisch veränderte Hefezelle nach einem der vorhergehenden Ansprüche in einem glycerolhaltigen Kulturmedium unter geeigneten Fermentationsbedingungen kultiviert und das Fermentationsprodukt von dem Kulturmedium abgetrennt wird.

11. Verfahren nach Anspruch 10, wobei die Hefezelle unter mikroaeroben, vorzugsweise unter anaeroben Bedingungen kultiviert wird.

12. Verfahren nach Anspruch 10 oder 11, wobei Glycerol das einzige Substrat und das Fermentationsprodukt eine C₄-Dicarbonsäure, vorzugsweise Succinat ist.

## Claims

1. A genetically modified yeast cell of the genus Saccharomyces for the fermentation of glycerol, wherein the yeast cell is genetically modified in such a way that
i) the degradation of glycerol into dihydroxyacetone phosphate via the glycerol-3 phosphate pathway is blocked,
ii) a heterologous glycerol uptake facilitator protein is expressed,
iii) a heterologous glycerol dehydrogenase catalysing the oxidation of glycerol into dihydroxyacetone is expressed or a native glycerol dehydrogenase catalysing the oxidation of glycerol into dihydroxyacetone is overexpressed,
iv) a heterologous dihydroxyacetone kinase is expressed or a native dihydroxyacetone kinase is overexpressed,
v) a heterologous C4-dicarboxylic acid transporter is expressed,
vi) a heterologous glycerol dehydratase is expressed,
vii) a heterologous 1,3-PDO oxidoreductase is expressed,
viii) a heterologous PEP carboxykinase is expressed or the native PEP carboxykinase is overexpressed, and
ix) the pyruvate carboxylase is deactivated.

2. The genetically modified yeast cell according to claim 1, wherein the yeast cell is additionally genetically modified in such a way that
x) a heterologous oxaloacetate decarboxylase is expressed or a native oxaloacetate decarboxylase is overexpressed; and/or
xi) a heterologous oxaloacetate decarboxylating malate enzyme is expressed or a native oxaloacetate decarboxylating malate enzyme is overexpressed.

3. The genetically modified yeast cell according to claim 2, wherein the yeast cell is additionally genetically modified in such a way that
xii1) a heterologous cytosolic malate dehydrogenase is expressed or the native cytosolic malate dehydrogenase is overexpressed; or
xii2) the cytosolic malate dehydrogenase is deactivated.

4. The genetically modified yeast cell according to one of claims 2 or 3, whereby the yeast cell is additionally genetically modified in such a way that
xiii) the pyruvate kinase is deactivated.

5. The genetically modified yeast cell according to one of the aforementioned claims, wherein the yeast cell is further genetically modified such that
xiv) a heterologous malate enzyme is expressed or the native malate enzyme is overexpressed.

6. The genetically modified yeast cell according to one of the aforementioned claims, wherein the yeast cell is a yeast cell of the species Saccharomyces cerevisiae.

7. The genetically modified yeast cell according to one of the aforementioned claims, whereby additionally
xv) a heterologous glycerol dehydratase reactivator is expressed; and/or
xvi) a heterologous fumarase is expressed; and/or
xvii) a heterologous fumarate reductase is expressed or the native fumarate reductase is overexpressed; and/or
xviii) the expression of the natural active glycerol transporter encoded by STLJ is blocked; and/or
xix) the synthesis pathway for the regeneration of glycerol from dihydroxyacetone phosphate is blocked; and/or
xx) the glyceraldehyde pathway is blocked.

8. The genetically modified yeast cell according to one of the preceding claims, wherein the degradation of glycerol into dihydroxyacetone phosphate via the glycerol-3-phosphate pathway is blocked by blocking the expression of glycerol kinase and/or mitochondrial glycerol-3-phosphate dehydrogenase.

9. The genetically modified yeast cell according to one of the aforementioned claims, wherein the heterologous glycerol uptake facilitator protein Fps2 from Pachysols tannophilus, the heterologous glycerol dehydrogenase is a glycerol dehydrogenase from Ogataea angusta, the heterologous C4-dicarboxylic acid transporter is a succinate transporter from Aspergillus niger and/or the heterologous 1,3-PDO oxidoreductase is from Clostridium butyricum.

10. A method for the preparation of a fermentation product from glycerol, wherein a genetically modified yeast cell according to one of the aforementioned claims is cultivated in a glycerol-containing culture medium under suitable fermentation conditions and the fermentation product is separated from the culture medium.

11. The method according to claim 10, wherein the yeast cell is cultivated under microaerobic, preferably anaerobic, conditions.

12. The method according to claim 10 or 11, wherein glycerol is the sole substrate and the fermentation product is a C4-dicarboxylic acid, preferably succinate.

## Revendications

1. Cellule de levure génétiquement modifiée du genre Saccharomyces pour la fermentation du glycérol, où la cellule de levure est génétiquement modifiée de telle sorte que
i) la dégradation du glycérol en phosphate de dihydroxyacétone par la voie du glycérol-3-phosphate est bloquée
ii) une protéine hétérologue facilitant l'absorption du glycérol est exprimée
iii) une glycérol déshydrogénase hétérologue catalysant l'oxydation du glycérol en dihydroxyacétone est exprimée ou une glycérol déshydrogénase native catalysant l'oxydation du glycérol en dihydroxyacétone est surexprimée,
iv) une dihydroxyacétone kinase hétérologue est exprimée ou une dihydroxyacétone kinase native est surexprimée,
v) un transporteur d'acide dicarboxylique C4 hétérologue est exprimé,
vi) une glycérol déshydratase hétérologue est exprimée
vii) une oxydoréductase 1,3-PDO hétérologue est exprimée,
viii) une PEP-carboxykinase hétérologue ou la PEP-carboxykinase native est surexprimée, et
ix) la pyruvate carboxylase est désactivée.

2. Cellule de levure génétiquement modifiée selon la revendication 1, **caractérisée en ce que** la cellule de levure est en outre génétiquement modifiée de telle manière que
x) une oxaloacétate décarboxylase hétérologue est exprimée ou une oxaloacétate décarboxylase native est surexprimée ; et/ou
xi) une enzyme de malate décarboxylant l'oxaloacétate hétérologue est exprimée ou une enzyme de malate décarboxylant l'oxaloacétate native est surexprimée.

3. Cellule de levure génétiquement modifiée selon la revendication 2, **caractérisée en ce que** la cellule de levure est en outre génétiquement modifiée de telle sorte que
xii1) une malate déshydrogénase cytosolique hétérologue est exprimée ou la malate déshydrogénase cytosolique native est surexprimée ; ou
xii2) la malate déshydrogénase cytosolique est désactivée.

4. Cellule de levure génétiquement modifiée selon l'une des revendications 2 ou 3, **caractérisée en ce que** la cellule de levure est en outre génétiquement modifiée de telle manière que xiii) la kinase pyruvate est désactivée.

5. Cellule de levure génétiquement modifiée selon l'une des revendications précédentes, où la cellule de levure est en outre génétiquement modifiée de telle sorte que xiv) une enzyme de malate hétérologue est exprimée ou l'enzyme de malate native est surexprimée.

6. Cellule de levure génétiquement modifiée selon l'une des revendications précédentes, où la cellule de levure est une cellule de levure de l'espèce Saccharomyces cerevisiae.

7. Cellule de levure génétiquement modifiée selon l'une des revendications précédentes, où en outre
xv) un réactivateur hétérologue de la glycérol déshydratase est exprimé ; et/ou
xvi) une fumarase hétérologue est exprimée ; et/ou
xvii) une fumarate réductase hétérologue est exprimée ou la fumarate réductase native est surexprimée ; et/ou
xviii) l'expression du transporteur de glycérol actif naturel codé par le STLJ est bloquée ; et/ou
xix) la voie de synthèse pour la reconstitution du glycérol à partir du phosphate de dihydroxyacétone est bloquée ; et/ou
xx) la voie du glycéraldéhyde est bloquée.

8. Cellule de levure génétiquement modifiée selon l'une des revendications précédentes, où la dégradation du glycérol en phosphate de dihydroxyacétone par la voie du glycérol-3-phosphate est bloquée en bloquant l'expression de la glycérol kinase et/ou de la glycérol-3-phosphate déshydrogénase mitochondriale.

9. Cellule de levure génétiquement modifiée selon l'une des revendications précédentes, où la protéine hétérologue facilitant l'absorption du glycérol Fps2 provient de Pachysols tannophilus, la glycérol déshydrogénase hétérologue est une glycérol déshydrogénase provenant de Ogataea angusta, le transporteur d'acide dicarboxylique en C4 hétérologue est un transporteur de succinate provenant de Aspergillus niger et/ou la 1,3-PDO oxydoréductase hétérologue provient de Clostridium butyricum.

10. Procédé de production d'un produit de fermentation à partir de glycérol, où une cellule de levure génétiquement modifiée selon l'une des revendications précédentes est cultivée dans un milieu de culture contenant du glycérol dans des conditions de fermentation appropriées et le produit de fermentation est séparé du milieu de culture.

11. Procédé selon la revendication 10, où la cellule de levure est cultivée dans des conditions microaérobies, de préférence anaérobies.

12. Procédé selon la revendication 10 ou 11, où le glycérol est le seul substrat et le produit de fermentation est un acide C4-dicarboxylique, de préférence un succinate.
